# EUROPEAN PATENT SPECIFICATION

(11) **EP 2 929 839 B1**
(45) Date of publication and mention of the grant of the patent: **20.02.2019**
(21) Application number: 12889675.0
(22) Date of filing: 07.12.2012
(51) Int. Cl.: B06B 1/02, G10K 11/34, G01S 15/89, G01S 7/52, A61B 8/00, B06B 1/06, G01N 29/26, A61B 8/14

(54) **ULTRASONIC PROBE AND ULTRASONIC DIAGNOSTIC APPARATUS**
ULTRASCHALLSONDE UND ULTRASCHALLDIAGNOSEVORRICHTUNG
SONDE À ULTRASONS ET DISPOSITIF DE DIAGNOSTIC ÉCHOGRAPHIQUE

(43) Date of publication of application: 14.10.2015
(73) Proprietor: Hitachi, Ltd., Tokyo 100-8280 (JP)
(72) Inventor: TANAKA, Hiroki, Chiyoda-ku Tokyo 100-8280 (JP); MASUZAWA, Hiroshi, Chiyoda-ku Tokyo 100-8280 (JP)
(74) Representative: Strehl Schübel-Hopf & Partner
(86) International application number: PCT/JP2012/081737
(87) International publication number: WO 2014/087532

(56) References cited:
- JP-A- S6 284 748
- JP-A- H01 214 347
- JP-A- H01 214 347
- JP-A- 2011 050 490
- US-A- 4 140 022
- US-A- 4 962 667
- US-B1- 6 468 216

## Description

### Technical Field

The present invention is a technology which relates to an ultrasonic probe and an ultrasonic diagnostic apparatus.

### Background Art

An ultrasonic diagnostic apparatus is configured to include a probe and an apparatus main body so as to image an internal structure of a living body and the like by using an ultrasonic wave. In an ultrasonic probe (the probe), a plurality of ultrasonic transducers (electro-acoustic conversion element) are built, thereby performing transmission and reception of an ultrasonic signal. As an ultrasonic transducer device, piezoelectric ceramic, single-crystal, a piezoelectric polymer, or a capacitive transducer is adopted. The devices generate an ultrasonic wave when being applied with a voltage and transmit the ultrasonic wave. In addition, the devices generate an electrical signal when an acoustic wave is received.

Generally, in the ultrasonic probe, the transducer is partitioned into multitudinous channels and is arrayed. When performing imaging, a delay time is appropriately applied to a transmission/reception signal of each channel, thereby generating an ultrasonic wave beam which is focused on a certain point.

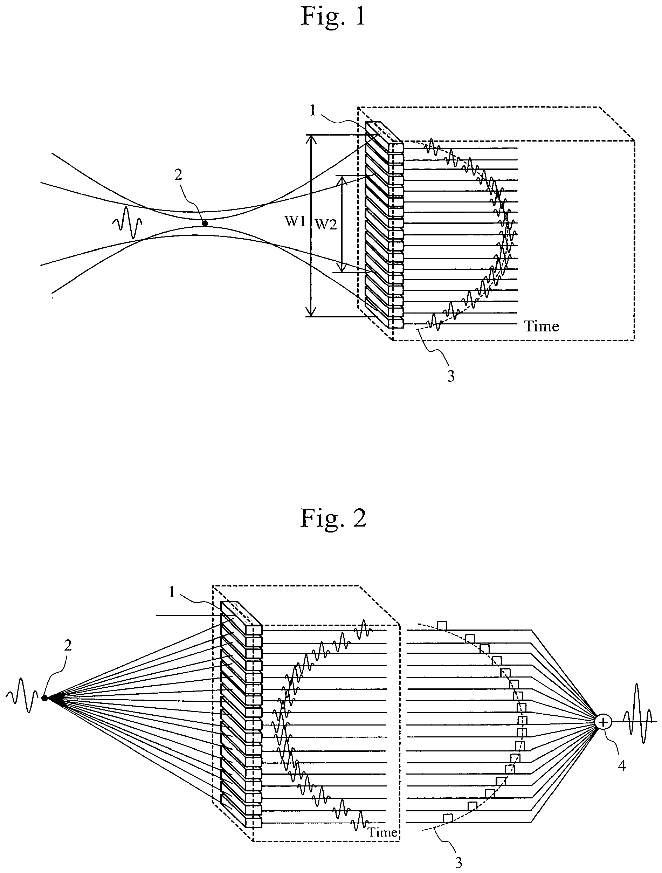

Fig. 1 illustrates forming of a transmission beam in the ultrasonic probe. Fig. 1 illustrates a state where an input signal applied with a delay time different from each other is applied to each channel of a one-dimensional array probe at the time of transmission. The ultrasonic probe in Fig. 1 includes a plurality of ultrasonic transducers 1. A delay circuit inside the ultrasonic probe or inside a main body apparatus applies a different delay time 3 to each channel of the ultrasonic transducer 1, thereby forming an ultrasonic wave beam focused on a focus point 2.

Fig. 2 illustrates forming of a reception beam in the ultrasonic probe. Fig. 2 illustrates a state where an echo signal is received in each channel of the one-dimensional array probe. The ultrasonic probe includes an adder 4 which is connected to the plurality of ultrasonic transducers 1. At the time of reception, the reception time for the echo signal received by each channel of the ultrasonic transducer 1 differs depending on a distance from the focus point 2. The delay circuit inside the ultrasonic probe or inside the main body apparatus applies the delay time 3 to reception signals of each channel in accordance with a propagation time difference, thereby arranging the phase. Each signal having the phase arranged is added by the adder 4 inside the ultrasonic probe or inside the apparatus, and the reception signal can be taken out as a signal which is focused on one point. A circuit which performs such processing is called a phasing circuit or a beamformer. When including adding, the circuit is called a phasing adder circuit.

The ultrasonic probe moves a focus point by changing the delay time, and acquires a signal of overall imaging region. The acquired signal is subjected to apodization processing, detection processing, filtering processing, and the like, thereby being displayed on a display of an ultrasonic diagnostic apparatus as an image.

Spatial resolution is one of the indexes for image quality of the ultrasonic diagnostic apparatus. Particularly, the spatial resolution in a lateral direction (an orientation direction) depends on the performance of focusing an ultrasonic wave beam. The spatial width of the focus point is determined by a frequency and the aperture. Therefore, when a width (a pitch) of one channel in the ultrasonic probe is fixed, the spatial resolution is improved as the allowed channels increase.

As illustrated in Fig. 1, a aperture W1 is greater than a aperture W2, and a beam can be focused further at the focus point 2. In addition, as the aperture increases, energy for transmitting and receiving an ultrasonic wave increases, and thus, signal to noise ratio (S/N) is also improved. However, there is a limitation in the number of the channels in a transmission beamformer and a reception beamformer of the apparatus main body of the ultrasonic diagnostic apparatus (generally, in an order of 10 channels to 200 channels) . Since the pitch of the channels is designed so as not to generate an artifact which is caused by the frequency in use, flexibility of design is low. Therefore, there is limitation in the aperture as well. Meanwhile, in a case of a matrix array in which the channels are arranged in a two-dimensional manner, the number of the channels to be handled in the ultrasonic probe may increase up to several thousands of the channel order, thereby leading to a situation in which the number of the channels of a main body beamformer is predominantly insufficient with respect to the number of the channels of the probe.

Therefore, it is considered that the plurality of channels inside the ultrasonic probe are collectively arranged so as to perform subarraying (PTL 1). Accordingly, the number of signal lines connected to the main body beamformer (a main beamformer) at the time of reception can be reduced (channel reduction) . In the method, separately from a main delay time in the main beamformer, after a small-delay time is added to each channel in the circuit at the previous stage, the plurality of channel signals are added. Accordingly, the circuit which realizes the above-mentioned performance is called "a micro-delay adder circuit" or "a micro-beamformer". Otherwise, the circuit is called "a sub-beamformer" and the like, while a main body circuit is called the main beamformer.

Meanwhile, when performing transmission according to the method, a voltage signal is distributed from one transmission line to the plurality of channels of the ultrasonic transducers, and thus, more channels can be handled within the limited number of the main body channels. Such a circuit is called "a micro-delay distribution circuit" and the like. On account of the sub-beamformer circuit for transmission/reception, even though the main body apparatus has the limited number of the channels, more probe channels can be handled. In addition, similarly to a two-dimensional matrix array, one thousand channels or more can be handled, and thus, three-dimensional volume imaging can be performed.

JP H01 214347 A discloses an ultrasonic diagnostic apparatus comprising a number of transducers and channels in which the reception signals are delayed separately from one another through variable delay lines. Also, US 4 962 667 A and US 4 140 022 A discloses ultrasonic imaging apparatuses with arrayed transducer elements the reception signals of which are subjected to varying delaying schemes.

### Citation List

### Patent Literature

PTL 1: JP-A-2005-270423

### Summary of Invention

### Technical Problem

However, generally, there are many cases where performance of a sub-beamformer such as a small-delay adder circuit and a delay distribution circuit is deteriorated compared to a main beamformer. The reason is that the circuits are restricted in design conditions of consumption power and the size when being inserted into a probe, compared to a case of being mounted in the main body apparatus. Even in a case where the circuits are inserted in the main body apparatus, the performance thereof is deteriorated further than that in the main beamformer, in order to achieve a cost benefit.

Currently, digitized main body beamformers (main beamformers) are the mainstream, and the delay time accuracy thereof is sufficiently high. In addition, since the main body beamformer is digitized, there is no S/N deterioration. Moreover, multiple-beam having a plurality of focus points at a time can be generated. Meanwhile, generally, the small-delay circuit (the sub-beamformer) is an analog delay circuit, and accuracy of the delay time and a noise level thereof are deteriorated further than those of the main body beamformer. In order to generate a multiple-beam, a plurality of small-delay circuits are necessary, thereby leading to an increase of the scale of the circuit. In a case of using the small-delay circuit, due to the limitation of consumption power and the like, it is not possible to realize sufficient number of multiple-beam similarly to the main body beamformer.

In order to perform subarraying, causing a signal to pass through a certain circuit which is deteriorated in beam forming performance compared to the main beamformer, such as the sub-beamformer, for example, a small-delay adder circuit and a small-delay distribution circuit leads directly to image deterioration. If accuracy of the delay time is insufficient, it is not possible to focus on a particular point inside an imaging space, thereby being deteriorated in resolution. Moreover, if S/N is insufficient, a weak signal is buried in noise, thereby being deteriorated in image depiction ability. When multiple ultrasonic wave beams are generated at a time, a frame rate can be increased or a high density image can be acquired. However, the small-delay adder circuit is unlikely to generate many beams as the main beamformer.

Regarding delay time accuracy, for example, when a signal of the frequency of 10 MHz is phased, if standard delay time accuracy in main body phasing is set to 1/64 wavelength (6 bit), the delay time accuracy of 1/10 MHz/64 = 1.5625 nsec is required. Depending on the purpose or the imaging condition of the superficial region, there is a case where a signal of the frequency up to 20 MHz is used. In such a case, twice the delay time accuracy is required. Therefore, delaying accuracy of the digital beamformer included in the currently used ultrasonic diagnostic apparatus exhibits time resolution of approximately 1 nsec. Meanwhile, in an analog delay method which is used in the small-delay adder circuit, due to various limited conditions, the time accuracy ranges approximately from 1/16 to 1/32 wavelength (4 bit to 5 bit) in many cases. In this case, the time accuracy ranges from 6.25 nsec to 12.5 nsec. Compared to an apparatus of the time accuracy of 1/64 wavelength in the related art, the circuit can be handled with sufficient accuracy only within a range of the wavelength of 1/4 to 1/2, that is, the frequency corresponding to 2.5 MHz to 5 MHz. Therefore, a signal passing through such a small-delay adder circuit has unfavorable focusing accuracy, thereby causing deterioration in resolution and S/N on an image, compared to that of the apparatus in the related art. Due to the similar reason, accuracy is deteriorated in a transmission small-delay distribution circuit as well compared to that of the main beamformer.

Generally, a signal from the ultrasonic transducer of the ultrasonic probe is amplified by a low noise amplifier (LNA), or is subjected to impedance conversion through a buffer circuit so as to increase S/N of the signal, thereby ensuring a necessary dynamic range. When being inserted into such an amplifier and a buffer circuit at the previous stage of the small-delay circuit, a certain amplification rate needs to be obtained in order to ensure a desired dynamic range. However, if the amplification rate is increased, consumption power of the circuit increases . As a result, the circuit generates heat. When such circuits are mounted inside the probe, another problem occurs in that the temperature standard of the probe cannot be fulfilled. Therefore, generally, the dynamic range is smaller than that is originally required.

As described above, causing a signal to pass through a circuit, that is, a small-delay adder circuit or a small-delay distribution circuit which is deteriorated further than the main beamformer built in the main body apparatus in the related art leads to image deterioration of an image in its entirety.

Therefore, the present invention provides a technology in which while the channel is increased and the aperture is widened, deterioration of image quality caused by the circuit for a subarray (the sub-beamformer) which exhibits less performance than the main body main beamformer is reduced.

### Solution to Problem

In order to solve the above-described problems, according to the present invention, channels of a transmission/reception main beamformer in a main body apparatus are divided into a channel group which passes through a sub beamformer such as a small-delay adder circuit and a small-delay distribution circuit, and a channel group which is connected directly to the main beamformer of the main body apparatus from a probe channel. The channel group of the probe which requires higher phasing accuracy or S/N is connected directly to the main beamformer, and the channel group of the probe which requires relatively less performance is connected to the main beamformer passing through the sub beamformer.

In order to solve the above-described problems, the ultrasonic diagnostic apparatus as defined in Claim 1 is suggested. Further advantageous features are set out in the dependent claims.

### Advantageous Effects of Invention

According to the present invention, image deterioration does not occur even though a circuit for a subarray (a sub-beamformer) which exhibits less performance compared to a main body main beamformer is used.

More characteristics related to the present invention are clarified through the disclosure of the present specification and the accompanying drawings. Problems, configurations, and effects other than those described above will be clarified through the descriptions of the following Examples.

### Brief Description of Drawings

Fig. 1 is a diagram illustrating forming of a transmission beam in an ultrasonic probe.
Fig. 2 is a diagram illustrating forming of a reception beam in the ultrasonic probe.
Fig. 3 is a configurational example of an ultrasonic diagnostic apparatus.
Fig. 4 is a configurational example of a subarray circuit.
Fig. 5 is a diagram illustrating distribution of amplitude of a reception signal in a probe channel direction.
Fig. 6 is a diagram illustrating a relationship between a focus depth and a aperture width.
Fig. 7 is a diagram illustrating a relationship between the focus depth and a spectrum of the reception signal.
Fig. 8 is a diagram illustrating distribution of apodization applied to a transmission/reception signal of a probe channel.
Fig. 9 is a diagram illustrating a reception system circuit in the ultrasonic diagnostic apparatus of First Example.
Fig. 10 is a diagram illustrating the reception system circuit in which 4:1 reduction and 9:1 reduction are adopted together.
Fig. 11 is a diagram illustrating a transmission system circuit in the ultrasonic diagnostic apparatus of First Example.
Fig. 12 is a diagram illustrating an example in which the transmission system circuit and the reception system circuit are not connected to the same subarray.
Fig. 13 is a diagram illustrating a configuration in which the probe channel is selected by using a channel selection switch.
Fig. 14 is a diagram illustrating another example of the configuration in which the probe channel is selected by using the channel selection switch.
Fig. 15 is a diagram illustrating the reception system circuit in the ultrasonic diagnostic apparatus of Second Example.
Fig. 16 is a diagram illustrating the ultrasonic probe in the ultrasonic diagnostic apparatus of Third Example and an application example with respect to a 1.25D matrix array and a 1.5D matrix array.
Fig. 17 is another diagram illustrating the ultrasonic probe in the ultrasonic diagnostic apparatus of Third Example and an application example with respect to a 2D matrix array.

### Description of Embodiments

Hereinafter, Examples of the present invention will be described with reference to the accompanying drawings. The accompanying drawings illustrate specific Examples conforming to the principle of the present invention. However, the Examples are provided so as to illuminate the present invention. The Examples are not to be adopted for interpreting the present invention in a limited manner.

### <First Example>

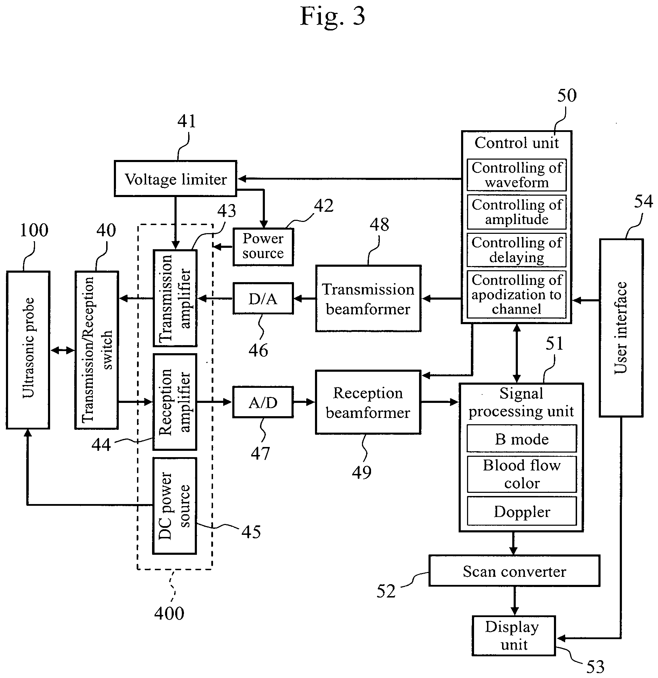

First, descriptions will be given regarding an apparatus configuration of an ultrasonic diagnostic apparatus and a flow of a signal up to a process of imaging the signal. Fig. 3 illustrates the apparatus configuration of a representative ultrasonic diagnostic apparatus.

The ultrasonic diagnostic apparatus includes an ultrasonic probe 100, a transmission/reception switch 40, transmission and reception system circuits 400, a voltage limiter 41, a power source 42, a DC power source 45, a D/A converter 46, an A/D converter 47, a transmission beamformer 48, a reception beamformer 49, a control unit 50, a signal processing unit 51, a scan converter 52, a display unit 53, and a user interface 54. As described below, the DC power source 45 is not necessarily included when connecting an ultrasonic probe which does not require a DC voltage.

The ultrasonic probe 100 in Fig. 3 corresponds to an ultrasonic transducer 1 which includes a plurality of channels illustrated in Figs. 1 and 2. Each channel of the ultrasonic probe 100 is switched between the transmission system circuit and the reception system circuit through the transmission/reception switch 40. The ultrasonic probe 100 operates as an array which forms an ultrasonic wave beam through a transmission amplifier 43 and a reception amplifier 44 driven by the power source 42. The ultrasonic probe 100 is utilized for transmitting and receiving an ultrasonic wave.

When the ultrasonic probe 100 requires a biased power source similar to a capacitive micro-machined ultrasonic transducer (CMUT), the ultrasonic probe 100 is connected to the DC power source 45.

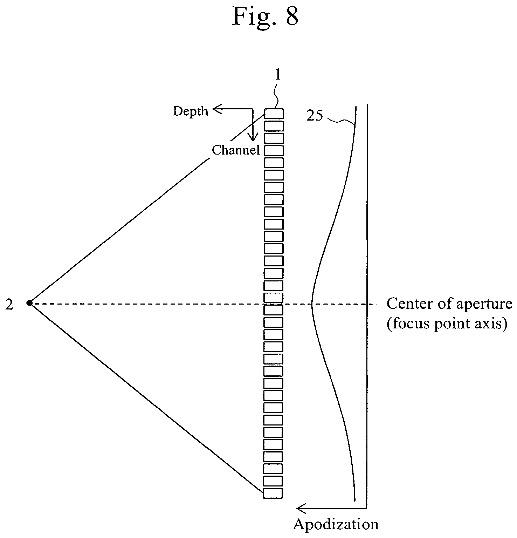

The plurality of channels of the ultrasonic probe 100 are connected to the transmission beamformer 48 and the reception beamformer 49 of an ultrasonic wave imaging device. A transmission/reception signal is controlled by the control unit 50 in accordance with an operation through the user interface 54. When transmitting a signal, the transmission signal is controlled by the control unit 50, and a waveform, amplitude, and a delay time are set to each channel. The control unit 50 may control apodization which is illustrated in Fig. 8. The transmission signal is transmitted to the ultrasonic probe 100 through the transmission beamformer 48, the D/A converter 46, and the transmission amplifier 43. Here, a voltage of which the waveform is formed due to controlling by the control unit 50 is input to the transmission amplifier 43, and the voltage is amplified through the transmission amplifier 43, thereby being output. Accordingly, a plurality of independent drive voltage signals for generating an ultrasonic wave are input to the plurality of channels of the ultrasonic probe 100. The voltage limiter 41 is provided in order to prevent a voltage from being excessively applied to the ultrasonic probe 100 or to control a transmission waveform.

When the ultrasonic probe 100 receives an ultrasonic wave signal, the reception signals in the plurality of channels are subjected to phasing (delaying) and adding. The reception signals are transmitted to the signal processing unit (an image processing unit) 51 after passing through the reception amplifier 44, the A/D converter 47, and the reception beamformer 49. The signal processing unit 51 executes B mode tomographic image processing or processing in accordance with the function thereof such as a blood flow color mode or Doppler, thereby converting the reception signal into a video signal. Thereafter, the video signal is transmitted to the display unit 53 through the scan converter 52, and the display unit 53 displays an image or a numerical value. The reception amplifier 44 is configured to be an LNA or a variable gain amplifier.

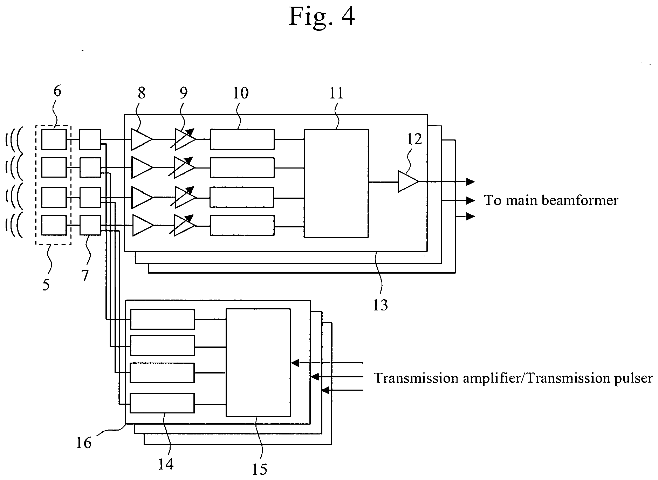

Subsequently, a general subarrayed circuit configuration will be described with reference to Fig. 4. Fig. 4 illustrates a configurational example of a subarray circuit in the ultrasonic diagnostic apparatus. The ultrasonic diagnostic apparatus includes a subarray reception circuit 13 and a subarray transmission circuit 16, as the subarray circuit.

A probe channel 6 in Fig. 4 corresponds to the probe channel of the ultrasonic transducer 1 illustrated in Figs. 1 and 2. In Fig. 4, the probe channels 6 of four ultrasonic transducers are configured as one subarray 5.

The subarray reception circuit 13 includes a low noise amplifier (LNA) 8, a variable gain amplifier (VGA) 9, a small-delay circuit 10, an adder circuit 11, and a buffer amplifier 12. Each of the reception signals acquired by some of the subarrayed probe channels 6 (four channels in Fig. 4) is added in the adder circuit 11 after passing through a transmission/reception separation circuit (or a protection circuit) 7, the LNA 8, the VGA 9, and the small-delay circuit 10, thereby becoming an electrical signal. Thereafter, the electrical signal from the adder circuit 11 passes through the buffer amplifier 12 and is transmitted to a main beamformer. As illustrated in Fig. 4, there are a plurality of such subarray circuits, and electrical signals (subarray signals) are transmitted to the main beamformer from the subarray circuit. In the main beamformer, delaying and adding are performed with respect to the plurality of subarray signals. Here, the LNA 8, the VGA 9, and the buffer amplifier 12 are appropriately used, and disposition sites thereof can vary.

The subarray transmission circuit 16 includes a small-delay circuit 14 and a distribution circuit 15. As for transmission, a high voltage signal from a transmission circuit such as a transmission amplifier and a transmission pulser is distributed to the plurality of channels through the distribution circuit 15, and the distributed signal is applied with a delay time in the small-delay circuit 14. Thereafter, the signal is transmitted to the probe channel 6. In a case of transmission, since focusing is not performed as much as that in a case of reception, the small-delay circuit 14 may not be inserted. When performing subarraying, the subarray reception circuit 13 and the subarray transmission circuit 16 are not necessarily mounted at the same time, and only one therebetween can be mounted.

In this manner, it is possible to handle the probe channels more than the number of the channels in the main beamformer by performing subarraying. For example, if the number of the probe channels included in one subarray is four, even though there are 48 channels in the main body main beamformer, signals of the probe channels (48 × 4 = 192 channels) can be handled.

However, as described above, when the performance (delay time accuracy, S/N, and the like) of subarraying the circuit is deteriorated further than that in the main beamformer which is built in the main body apparatus in the related art, the deterioration affects an image in its entirety. A signal which is added with unfavorable small-delay accuracy in the small-delay circuit cannot be restored to the signal state before being added in the main body apparatus. Therefore, even if the main beamformer of the main body apparatus has high delay time accuracy, the delay time accuracy is determined eventually by the accuracy of a small-delay adder circuit. Otherwise, degradation of S/N and a dynamic range may occur depending on the performance of an LNA or a VGA. In the circuit of Fig. 4, the VGA 9 is inserted into each of the probe channels 6. However, the VGA 9 may not be able to be inserted into all the channels due to consumption power and the like. When a VGA is inserted after being added, only discontinuous (coarse) apodization can be applied to each the subarray signal. In this case, block noise may be caused on an image.

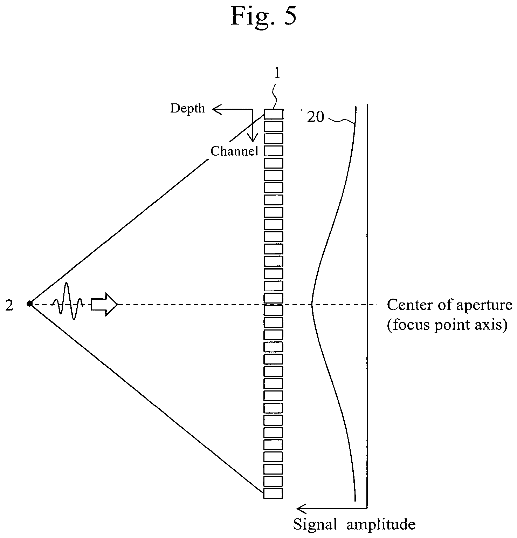

Therefore, the present invention focuses on three characteristics which appear when an ultrasonic diagnostic machine performs imaging. The first characteristic will be described with reference to Fig. 5. Fig. 5 illustrates distribution 20 of amplitude of a reception signal in a probe channel direction when an echo signal reflected from a certain focus point 2 is received in a one-dimensional linear array probe.

Since the channels in the vicinity of the center of a aperture (the axis of a focus point) are positioned in front of a beam transmitted toward a reflector and has the shortest distance therebetween, acoustic energy is concentrated and the amplitude of a signal becomes a maximum. Therefore, in a process of imaging, the influence degree with respect to an image quality of a reception signal of the channels in the vicinity of the center of the aperture (the axis of the focus point) is high, and the influence degree of a reception signal of the channel away from the center of the aperture is low. In other words, it is desirable that the channel closer to the center of the aperture includes a signal having higher delay time accuracy and S/N.

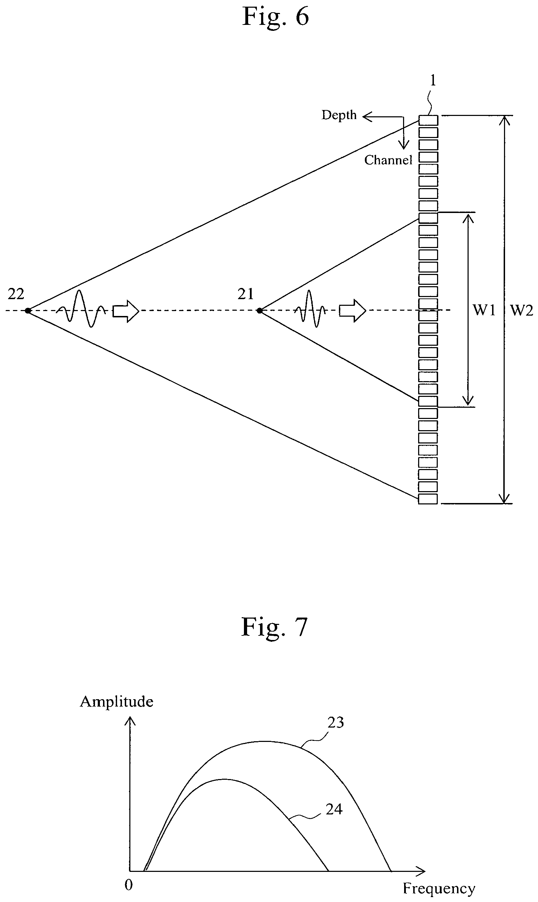

The second characteristic will be described with reference to Figs. 6 and 7. Fig. 6 is a diagram illustrating a relationship between a focus depth and a aperture width, and Fig. 7 is a diagram illustrating a relationship between the focus depth and a spectrum of a reception signal.

Generally, in the ultrasonic diagnostic apparatus, the width of a aperture to be used is changed in accordance with the depth to be imaged. Practically, a aperture ratio (f-number = focus distance / aperture) is substantially fixed and used. It is because a focus region in a depth direction is to be limited to a certain extent. Therefore, for example, in Fig. 6, the aperture of a focus point 21 at a superficial portion is referred to as W1, and the aperture of a focus point 22 at a deep portion is referred to as W2. In other words, as the focus point is closer to the superficial portion, only the signals of the channels in the vicinity of the center of the aperture are used, and as imaging of a deeper position is performed, signals of the channel away from the center of the aperture are utilized. The channels in the vicinity of the center of the aperture are used at all times. However, as the channel is farther away from the center of the aperture, a frequency of use decreases.

Incidentally, there is attenuation in distance as one of the characteristics of an ultrasonic wave. The characteristic is that as a propagated distance becomes longer, the ultrasonic wave is attenuated, and as the frequency becomes higher, an amount of attenuation increases. Fig. 7 illustrates a signal spectrum 23 of the focus point at the superficial portion, and a signal spectrum 24 of the focus point at the deep portion.

As illustrated in Fig. 7, the signal spectrum 23 at the superficial portion is less attenuated and includes an even a higher frequency signal. Meanwhile, the signal spectrum 24 of the deep portion is attenuated in all the frequencies. The signal spectrum 24 is attenuated further on a side of a relatively higher frequency, and a center frequency (bandwidth/2) becomes a low frequency. In other words, the aperture becomes narrow at the superficial portion, and even signals on the high frequency side need to be handled. The aperture becomes wide at the deep portion, and signals of a low frequency are dominant. As described above, as a frequency becomes high, delay time accuracy is required, and as a frequency becomes low, accuracy may be coarse. Therefore, in a process of imaging, the channel closer to the vicinity of the center of the aperture requires highly accurate delay time accuracy, and the channel away from the center of the aperture may have relatively low delay time accuracy.

Subsequently, the third characteristic will be described with reference to Fig. 8. Fig. 8 illustrates distribution 25 of apodization applied to the transmission/reception signal of each channel in a probe when the probe opens a certain aperture.

In an ultrasonic diagnostic machine, in order to improve the characteristics of an ultrasonic wave beam, apodization is applied in a channel direction so as to prevent signal strength from varying discontinuously at an end portion of the aperture. Apodization to the channel at the center of the aperture is great, and apodization to the channel away from the center of the aperture is small. Therefore, the influence degree of a signal of the channel away from the center of the aperture becomes relatively low, and an influence with respect to an image becomes small as much as thereof.

Taking the above-described three characteristics into account, the following configuration is considered in the present invention. The channels of the main beamformer for transmission and reception in the main body apparatus are divided into a channel group which passes through a sub beamformer such as the small-delay adder circuit and a small-delay distribution circuit, and a channel group which is connected directly to the main beamformer of the main body apparatus from the probe channel.

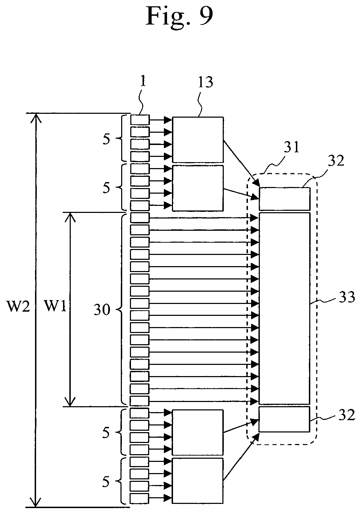

Regarding a case of a one-dimensional array as an example, Fig. 9 illustrates a configurational example thereof. Fig. 9 illustrates the reception system circuit in the ultrasonic diagnostic apparatus of First Example. In Fig. 9, the probe channels of four ultrasonic transducers 1 are configured as one subarray 5. The subarray reception circuit 13 in Fig. 9 corresponds to the subarray reception circuit 13 in Fig. 4. In other words, the subarray reception circuit 13 is a delay adder circuit in which the probe channels of four ultrasonic transducers 1 are configured as one subarray, and delaying and adding are performed in subarray units with respect to an ultrasonic wave reception signal acquired from the ultrasonic transducer 1 included in the subarray 5.

A main body beamformer 31 is a delay adder circuit in which delaying and adding are performed with respect to an ultrasonic wave reception signal acquired from the ultrasonic transducer 1 and corresponds to the main beamformer for reception. In the example of Fig. 9, the channels included in the main body beamformer 31 are divided into two groups. The channels of the main body beamformer 31 are divided into a first main body beamformer channel 32 and a second main body beamformer channel 33.

In the present Example, a plurality of the ultrasonic transducers 1 include a first group and a second group. The first group is a group which forms the subarrays 5 inside the probe channel of the ultrasonic transducer 1 and is connected to the first main body beamformer channel 32 passing through the subarray reception circuit 13. The second group is a group which is connected directly to the second main body beamformer channel 33 from probe channels 30 of the ultrasonic transducer 1.

In the above-described example, it is desirable that the probe channels 30 which are connected to the second main body beamformer channel 33 are concentrated in the vicinity of the center of the aperture, and the subarrayed group is disposed in a region away from the center of the aperture. In the example of Fig. 9, the probe channels 30 which are connected directly to the second main body beamformer channel 33 are continuously disposed in the vicinity of the center of the aperture. Meanwhile, the probe channels at both ends being away from the center of the aperture are configured as the subarrays 5, and are connected to the first main body beamformer channel 32 passing through the subarray reception circuit 13.

Therefore, in a case of a aperture W1, a reception signal is transmitted directly to the second main body beamformer channel 33. In addition, in a case of a aperture W2, a reception signal in the vicinity of the center of the aperture is transmitted directly to the second main body beamformer channel 33, and a reception signal at a position away from the center of the aperture is transmitted to the first main body beamformer channel 32 passing through the subarray reception circuit 13. Accordingly, signals which are used at all times and are in the vicinity of the center of the aperture requiring delay time accuracy and S/N are directly processed in the main body beamformer 31 (the main beamformer) with high accuracy and high sensitivity, thereby generating a signal which becomes a high-resolution image coming close to an apparatus in the related art. Meanwhile, signals of the channel away from the center of the aperture are relatively low in a frequency of use. Otherwise, even in a case of being used, apodization to the signal is relatively small, and thus, an influence degree with respect to an image is low. Moreover, even though the delay time accuracy of the subarray reception circuit 13 is low, when the aperture is in a significantly open state, the frequency band to be used becomes small, and thus, an influence on an image is small. In other words, according to the present Example, even though a signal which is deteriorated due to the subarray reception circuit 13 is generated, an image can be prevented from deteriorating.

In the subarraying of the related art, since all the channels inside the aperture are completely subarrayed and all the signals are deteriorated, thereby leading to image deterioration of an image in its entirety. However, in the present Example, taking into consideration that not all the channel signals have the uniform characteristics, the number of the channels of the main body beamformer 31 is divided into multiple numbers. Then, the probe channel which is subjected to higher apodization and requires accuracy is connected directly to the main body beamformer 31, and the probe channel which allows relatively low performance is subarrayed. Accordingly, an image can be prevented from deteriorating.

According to the present Example, when the number of the channels in the main beamformer counts 64 channels, and the small-delay adder circuit for reducing the channels from four channels to one channel (4:1 reduction) is adopted, for example, the probe channels of 44 channels among 64 channels are connected directly to the main beamformer, and are disposed at the center of the aperture. The remaining 20 channels are set for the subarray, thereby disposing the channels (20 channels × 4 = 80 channels) by 40 channels at both ends away from the center of the aperture. Therefore, equivalently, the probe including the aperture of 124 channels is obtained. According to such a configuration, even though the small-delay adder circuit and the like exhibiting less performance are used, the aperture can be widened without deteriorating an image.

Naturally, when a circuit having a reduction ratio of 9:1, 16:1, and the like in place of the reduction ratio of 4:1 is adopted, the number of the probe channels which can be equivalently handled with a fewer number of channels in the main body can be increased. The reduction ratio is not particularly limited, and any ratio may be adopted in accordance with the design condition.

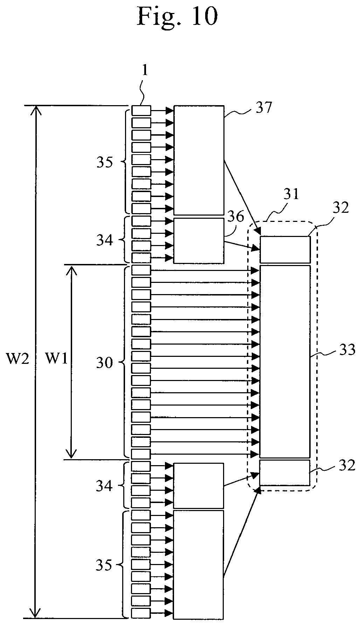

In the above-described example, the reduction ratio of the subarray is fixed, but the subarray may have multiple reduction ratios at the same time. For example, Fig. 10 illustrates an example in which 4:1 reduction and 9:1 reduction are adopted together. In Fig. 10, four probe channels are configured as a first subarray 34, and nine probe channels are configured as a second subarray 35. The probe channels of the first subarray 34 are connected to the first main body beamformer channel 32 passing through a circuit 36 for the first subarray. In addition, the probe channels of the second subarray 35 are connected to the first main body beamformer channel 32 passing through a circuit 37 for the second subarray. The first and second circuits 36 and 37 for subarrays correspond to the subarray reception circuits 13 in Fig. 4. In Figs. 9 and 10, the pattern of the subarray is bilaterally symmetrical with respect to the center of the aperture. However, the pattern of the subarray is not necessarily bilaterally symmetrical with respect to the center of the aperture.

In the above-described example, 44 channels among 64 channels which are the number of the channels included in the main beamformer are connected directly to the main beamformer as the probe channels for direct connection. However, the distribution is not necessarily uniform, and may vary in accordance with the situation. For example, depending on the design, 32 channels among 64 channels may be used as the probes for direct connection, and the remaining 32 channels may be used for the subarray.

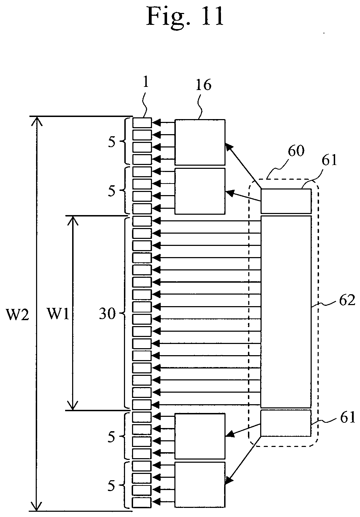

The similar configuration is applied to the case of transmission as well. Fig. 11 illustrates the transmission system circuit in the ultrasonic diagnostic apparatus of First Example, which in itself is not an embodiment of the present invention. In Fig. 11, the probe channels of four ultrasonic transducers 1 are configured as one subarray 5. The subarray transmission circuit 16 in Fig. 11 corresponds to the subarray transmission circuit 16 in Fig. 4. In other words, the subarray transmission circuit 16 configures the four ultrasonic transducers 1 as one subarray 5, and includes a transmission distribution circuit in which distribution is performed in subarray units with respect to the drive voltage signal in order to generate an ultrasonic wave from the ultrasonic transducer 1 included in the subarray 5, and the small-delay circuit which applies a delay time to distributed signals. As described above, the subarray transmission circuit 16 may not include the small-delay circuit.

A transmission circuit 60 is a circuit for transmission through which the plurality of independent drive voltage signals for generating an ultrasonic wave are transmitted from the ultrasonic transducer 1, and corresponds to the main beamformer for transmission. In the example of Fig. 11, the channels included in the highly accurate transmission circuit 60 are divided into two groups. The channels of the transmission circuit 60 are divided into a first main body beamformer channel 61 and a second main body beamformer channel 62.

In the present Example, the plurality of ultrasonic transducers 1 include the first group and the second group. The first group is a group in which the drive voltage signal is input from the first main body beamformer channel 61 to the probe channel passing through the subarray transmission circuit 16 which performs delaying distribution. The second group is a group in which the drive voltage signal is input directly from the second main body beamformer channel 62 to the probe channel.

In this manner, the number of the channels of the transmission circuit 60 is divided into multiple numbers. Then, the probe channels (the probe channels in the vicinity of the center of the aperture) which are subjected to higher apodization and require accuracy are connected directly to the second main body beamformer channel 62, and the probe channels (the probe channels away from the center of the aperture) which allow relatively low performance are subarrayed. According to such a configuration, even though the small-delay distribution circuit and the like exhibiting less performance are used, the aperture can be widened without deteriorating an image.

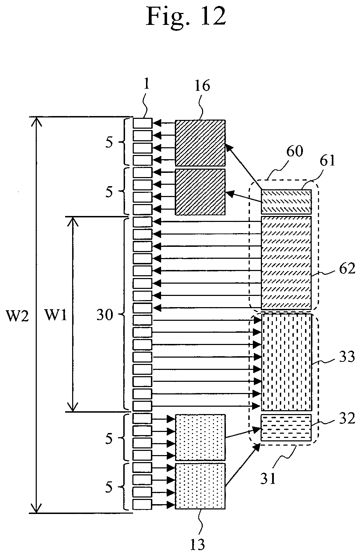

As illustrated in Fig. 3, since there is a method of selecting the probe channel by using the transmission/reception switch 40 and the like, the transmission system circuit and the reception system circuit are not necessarily connected to the same subarray. Fig. 12 illustrates an example in which the transmission system circuit and the reception system circuit are not connected to the same subarray. In the example of Fig. 12, a predetermined number among the probe channels 30 in the vicinity of the center of the aperture is connected directly to the second main body beamformer channel 33. Meanwhile, the probe channels at one end portion away from the center of the aperture are configured as the subarray 5, and are connected to the first main body beamformer channel 32 passing through the subarray reception circuit 13. In addition, the remaining channels among the probe channels 30 in the vicinity of the center of the aperture are connected directly to the second main body beamformer channel 62. Meanwhile, the probe channels at the other end portion away from the center of the aperture are connected to the first main body beamformer channel 61 passing through the subarray transmission circuit 16.

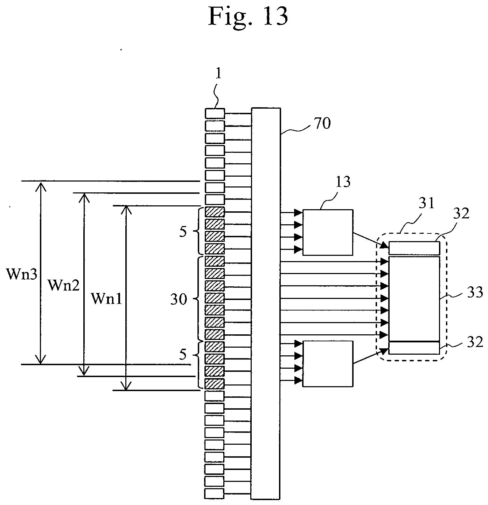

Fig. 13 illustrates a configuration in which the probe channel is selected by using a channel selection switch. Fig. 13 illustrates the reception system circuit as an example, and the transmission system circuit can have the same configuration.

In Fig. 13, a channel selection switch 70 is provided between the reception system circuit (the subarray reception circuit 13 and the main body beamformer 31) and the ultrasonic transducer 1. The channel selection switch 70 is utilized for moving the aperture to be used, and is controllable by the control unit 50 and the like in Fig. 3. The channel selection switch 70 allows selection to be arbitrarily made between the ultrasonic transducer 1 which is selected as the subarray 5, and the ultrasonic transducer 1 which is selected as the probe channels 30 which are connected directly to the second main body beamformer channel 33.

By using the channel selection switch 70, the aperture can be appropriately moved and used in a similar manner as the so-called linear scanning. For example, if a aperture which is used when acquiring data of a certain raster on an image is referred to as Wn1, a aperture of Wn2 is used in the next raster, and Wn3 is used in the successive raster thereafter. Since selection of the channels is freely made, the channels are not necessarily moved one by one when moving the aperture. Moreover, the patterns of the probe channel which is connected directly to the main body beamformer 31 (the main beamformer) and the probe channel which is connected to the subarray reception circuit 13 (the sub beamformer) may be appropriately changed.

By using the channel selection switch 70, for example, a pattern in which nearly all the channels of the main beamformer are connected directly to the ultrasonic transducer 1 may be formed. Compared to a case where the subarray is used, even though the aperture is limited, the pattern is suitable for a case where imaging is performed at a high resolution of an image. On the contrary, the subarraying ratio can be increased so as to put priority on imaging at the aperture.

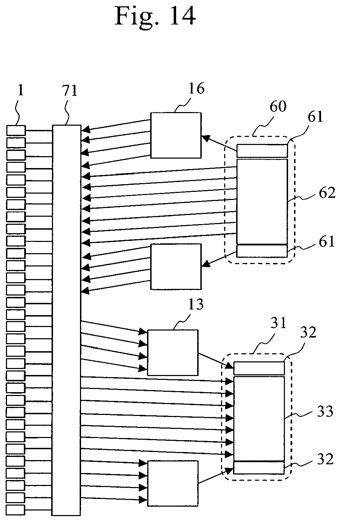

Fig. 14 illustrates another example of the configuration in which the probe channel is selected by using the channel selection switch. In Fig. 14, a channel selection switch 71 is provided between the reception system circuit (the subarray reception circuit 13 and the main body beamformer 31) and the transmission system circuit (the subarray transmission circuit 16 and the transmission circuit 60), and the ultrasonic transducer 1. The channel selection switch 71 allows selection of the probe channel, and also performs switching between transmission and reception.

By using the channel selection switch 71, as illustrated in Fig. 14, the probe channel can be freely selected to be used for transmission and reception. Accordingly, the aperture which is used at the time of transmission and the aperture which is used at the time of reception are arbitrarily changed so that optimal selection of the channel and setting of the aperture can be performed in both transmission and reception.

In a case where the number of the channels in the main beamformer is excessively few, and the like, the aperture divided in the main beamformer becomes small, and at a glance, the present invention may be considered to lose effectiveness. However, the effectiveness of the present invention can be retained by using an imaging technology such as a synthetic aperture and the like. In the synthetic aperture, even though the aperture width which can be handled at a time is limited, transmission and reception are performed multiple times by changing the aperture position. Then, a plurality of acquired items of information are collectively arranged afterwards as one, and thus, a captured image can be configured to be the same as if data is acquired through a large aperture. The aperture to be used can be realized by using the channel selection switches 70 and 71. The channel selection switches 70 and 71 may be provided in the ultrasonic probe 100.

### <Second Example>

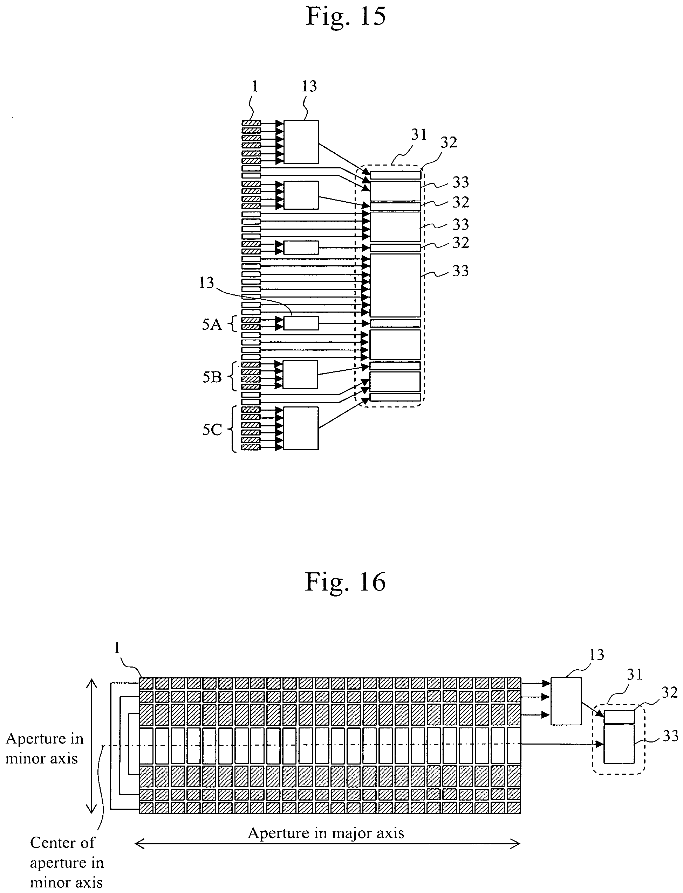

In First Example, the probe channels connected to the main beamformer are continuously disposed in the vicinity of the center of the aperture, and the subarrays are continuously disposed at positions away from the aperture. However, when the difference in the performance between the main beamformer and the sub beamformer is excessively significant, there is a possibility that an unfavorable influence is discontinuously caused in the image quality as soon as the aperture enters the subarray region while performing imaging. Fig. 15 illustrates the reception system circuit in the ultrasonic diagnostic apparatus of Second Example which is provided in order to solve such a problem. Fig. 15 illustrates the reception system circuit as an example, and the transmission system circuit can have the same configuration.

In Fig. 15, the channels of the main body beamformer 31 are divided into a plurality of the first main body beamformer channels 32 and a plurality of the second main body beamformer channels 33. The group which is disposed in the vicinity of the center of the aperture among the probe channels of the ultrasonic transducer 1 is connected directly to the second main body beamformer channels 33. In addition, two probe channels adjacent to the channel are configured as a subarray 5A and are connected to the first main body beamformer channels 32 passing through the subarray reception circuit 13. Moreover, the group of the probe channels adjacent to the subarray 5A is connected directly to the second main body beamformer channel 33.

In this manner, the group of the probe channels connected directly to the second main body beamformer channels 33, and the group of the subarrayed probe channels are alternately disposed. In the example of Fig. 15, since the group of the probe channels connected directly to the second main body beamformer channels 33, and the group of the subarrayed probe channels are discontinuously disposed, it is possible to avoid an influence on the image quality as soon as the aperture enters the subarray region while performing imaging.

As for being away from the center of the aperture, it is desirable that the number of the channels (the aperture width) connected directly to the main body beamformer 31 is gradually decreased, and on the contrary, the region of the subarray is gradually increased. In the example of Fig. 15, as being away from the center of the aperture, the number of the channels (the aperture width) connected directly to the second main body beamformer channels 33 is gradually decreased, and the regions of the subarrays 5A, 5B, and 5C are gradually increased. By having such a configuration, it is possible to be smoothly connected to a region where the sub beamformer is used, and it is possible to avoid discontinuity in an image.

### <Third Example>

The first and second embodiments described above presume the one-dimensional array. However, the present invention can also be applied to a matrix array. Fig. 16 is a configurational example of a 1.25D array and a 1.5D array. The 1.25D denotes a matrix array in which the aperture of the probe in the minor axis is variable, and the 1.5D denotes a matrix array in which the focus point on the minor axis side can be arbitrarily set on the central axis of the aperture in the minor axis.

Fig. 16 illustrates a structure of an array seen from above an acoustic radiation plane of the ultrasonic probe. In the ultrasonic probe of Fig. 16, the channels of the probe are divided not only in a major axis direction but also in a minor axis direction. Since an ultrasonic wave beam on a cross section of 1.25D and 1.5D in the minor axis is symmetric to the center of the aperture in the minor axis, the channels on both sides are applied with short circuits and the same delay time or apodization.

Regarding imaging, acoustic energy is mostly concentrated in the vicinity of the center of the aperture in the minor axis. In addition, apodization with respect to the transmission/reception signal of the channel away from the center of the aperture in the minor axis is decreased, or when imaging the vicinity, the aperture is narrowed in the minor axis so as to be used. Therefore, similar to the aperture in the major axis, in the aperture in the minor axis as well, it is desirable that the channels in the vicinity of the center of the aperture have signals having higher delay time accuracy and higher S/N.

In the present Example as well, the channel of the main body beamformer 31 includes the first main body beamformer channel 32 and the second main body beamformer channel 33. In the matrix array of the present Example, the channels in the vicinity of the center of the aperture in the minor axis are connected directly to the second main body beamformer channel 33, and the channel group on the periphery of the center of the aperture in the minor axis is connected to the first main body beamformer channel 32 passing through the subarray reception circuit 13.

In 1.75D, a beam is tilted in the minor axis direction as well (not illustrated), both sides of the channel in the minor axis are not short-circuited, thereby applying different delay time. Therefore, an independent sub beamformer is connected to the probe channels on both sides of the center of the aperture in the minor axis. Depending on distribution of the number of the channels in the main beamformer, the subarray region may be provided even for the channel at the center of the aperture in the minor axis, thereby applying the same configuration as the one-dimensional array. In Fig. 16, only the reception system circuit is illustrated. However, the matrix array can also be applied to the transmission system circuit.

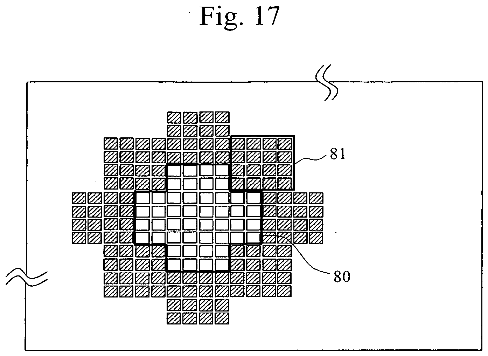

Fig. 17 is a configurational example of a 2D matrix array in which the number of division in the minor axis is increased. The 2D matrix array has a structure in which the focus point is arbitrarily formed in both the major axis direction and the minor axis direction. Generally, in the 2D matrix array, since the aperture ratio of the minor axis and the aperture ratio of the major axis come close to each other, the center of the aperture mostly has a shape which is close to a circle or a square.

In the present Example, as illustrated in Fig. 17, the probe channels of the ultrasonic transducer are divided into a channel group 80 which is connected directly to the main beamformer, and a channel group 81 which forms one subarray and is connected to the main beamformer passing through the sub beamformer. In Fig. 15, the group connected directly to the main beamformer, and the subarray group are continuously disposed. However, as the case of the one-dimensional array illustrated in Fig. 15, the groups are not necessarily continuous, and the disposition pattern thereof may be arbitrarily set by using the channel selection switch and the like.

In the case of the 2D matrix array, the number of the channels in the main beamformer reduces remarkably with respect to the number of the channels (several thousands of the channel order) which the ultrasonic probe physically includes. Therefore, the aperture area formed in the channel connected directly to the main beamformer may not be sufficiently secured. However, as described above, by using a method of aperture synthesis and the like, it is possible to obtain an image which is imaged by an ultrasonic wave beam having the equivalently significant aperture through multiple times of transmission and reception. Therefore, in the 2D matrix array as well, the effectiveness of the present invention is not affected in the least.

The present invention is not limited to Examples described above and includes various deformation examples. For example, Examples described above are given in order to illuminate the present invention in detail. However, the present invention is not limited to Example including all the described configurations. In addition, a portion of the configuration of one Example can be replaced with the configuration of another Example, and the configuration of one Example may be added to the configuration of another Example. With respect to a portion of the configuration of each Example, another configuration can be added, omitted, and replaced. For example, in the ultrasonic diagnostic apparatus, when performing subarraying, the subarray reception circuit 13 and the subarray transmission circuit 16 are not necessarily mounted at the same time, and only one therebetween can be mounted.

### Reference Signs List

1 ULTRASONIC TRANSDUCER
2 FOCUS POINT
3 DELAY TIME
4 ADDER
5 SUBARRAY
6 PROBE CHANNEL
7 TRANSMISSION/RECEPTION SEPARATION CIRCUIT OR PROTECTION CIRCUIT
8 LNA (LOW NOISE AMPLIFIER)
9 VGA (VARIABLE GAIN AMPLIFIER)
10 SMALL-DELAY CIRCUIT (FOR RECEPTION)
11 ADDER CIRCUIT
12 BUFFER AMPLIFIER
13 SUBARRAY RECEPTION CIRCUIT (SUB BEAMFORMER FOR RECEPTION)
14 SMALL-DELAY CIRCUIT (FOR TRANSMISSION)
15 DISTRIBUTION CIRCUIT
16 SUBARRAY TRANSMISSION CIRCUIT (SUB BEAMFORMER FOR TRANSMISSION)
20 AMPLITUDE DISTRIBUTION OF RECEPTION SIGNALS (DISTRIBUTION IN CHANNEL DIRECTION)
21 FOCUS POINT OF SUPERFICIAL PORTION
22 FOCUS POINT OF DEEP PORTION
23 SIGNAL SPECTRUM OF SUPERFICIAL PORTION
24 SIGNAL SPECTRUM OF DEEP PORTION
30 GROUP DIRECTLY CONNECTED TO MAIN BODY BEAMFORMER
31 MAIN BODY BEAMFORMER (MAIN BEAMFORMER)
32 FIRST MAIN BODY BEAMFORMER CHANNEL OF RECEPTION MAIN BEAMFORMER CHANNEL
33 SECOND MAIN BODY BEAMFORMER CHANNEL OF RECEPTION MAIN BEAMFORMER CHANNEL
34 FIRST SUBARRAY (4 CHANNELS SUBARRAY)
35 SECOND SUBARRAY (9 CHANNELS SUBARRAY)
36 FIRST CIRCUIT FOR SUBARRAY (4:1 REDUCTION CIRCUIT)
37 SECOND CIRCUIT FOR SUBARRAY (9:1 REDUCTION CIRCUIT)
60 TRANSMISSION CIRCUIT (MAIN TRANSMISSION BEAMFORMER (AMPLIFIER))
61 FIRST MAIN BODY BEAMFORMER CHANNEL OF TRANSMISSION MAIN BEAMFORMER
62 SECOND MAIN BODY BEAMFORMER CHANNEL OF TRANSMISSION MAIN BEAMFORMER
70 CHANNEL SELECTION SWITCH
71 CHANNEL SELECTION SWITCH
80 CHANNEL GROUP DIRECTLY CONNECTED TO MAIN BEAMFORMER IN 2D ARRAY
81 CHANNEL GROUP PASSING THROUGH SUB BEAMFORMER IN 2D ARRAY (4 CHANNELS × 4 CHANNELS = 16 CHANNELS SUBARRAY)
100 ULTRASONIC PROBE
400 TRANSMISSION AND RECEPTION SYSTEM CIRCUIT

## Claims

1. An ultrasonic diagnostic apparatus comprising:
an ultrasonic probe (100) having a plurality of ultrasonic transducers (1) in which a predetermined number among the plurality of ultrasonic transducers is configured as at least one subarray (5, 34, 35), thereby defining a first group of ultrasonic transducers,
at least one first delay adder circuit (13, 36, 37) configured to perform delaying and adding in the at least one subarray (5, 34, 35), on reception signals acquired from the ultrasonic transducers included in the at least one subarray, and
a second delay adder circuit (31) configured to perform delaying and adding on reception signals acquired from the plurality of ultrasonic transducers,
wherein the plurality of ultrasonic transducers include:
the first group configured to transmit the reception signals (32), that are processed by the first delay adder circuit (13, 36, 37), to the second delay adder circuit, and
a second group configured to transmit the reception signals (33) directly to the second delay adder circuit without passing through the first delay adder circuit, **characterized in that** the ultrasonic transducers of the second group are continuously disposed in the vicinity of the center of an aperture through which an ultrasonic wave beam is formed.

2. The ultrasonic diagnostic apparatus according to Claim 1,
wherein the first group includes a plurality of the subarrays (5) for each of which the number of the ultrasonic transducers (1) included in the subarray is different from one another.

3. The ultrasonic diagnostic apparatus according to Claim 1,
wherein the ultrasonic transducers (1) included in the subarray (5) of the first group and the ultrasonic transducers included in the second group are alternately disposed.

4. The ultrasonic diagnostic apparatus according to Claim 1,
wherein the ultrasonic probe (100) forms a matrix array which is divided along a major axis and a minor axis.

5. The ultrasonic diagnostic apparatus according to Claim 1, further comprising:
an ultrasonic transducer selection switch (70, 71) adapted to arbitrarily select between the ultrasonic transducers (1) included in the first group and the ultrasonic transducers included in the second group.

## Patentansprüche

1. Ultraschalldiagnosevorrichtung, umfassend:
eine Ultraschallsonde (100) mit mehreren Ultraschalltransducern (1), in der eine vorgegebene Anzahl aus den mehreren Ultraschalltransducern als mindestens eine Unteranordnung (5, 34, 35) eingerichtet ist, wodurch eine erste Gruppe von Ultraschalltransducern definiert wird,
mindestens eine erste Verzögerungsadditionsschaltung (13, 36, 37), die dazu eingerichtet ist, Verzögern und Addieren in der mindestens einen Unteranordnung (5, 34, 35) an Empfangssignalen auszuführen, die von den in der mindestens einen Unteranordnung enthaltenen Ultraschalltransducern erfasst worden sind, und
eine zweite Verzögerungsadditionsschaltung (31), die dazu eingerichtet ist, Verzögern und Addieren an Empfangssignalen, die von den mehreren Ultraschalltransducern erfasst worden sind, auszuführen,
wobei die mehreren Ultraschalltransducer enthalten:
die erste Gruppe, die dazu eingerichtet ist, die Empfangssignale (32), die von der ersten Verzögerungsadditionsschaltung (13, 36, 37) verarbeitet werden, an die zweite Verzögerungsadditionsschaltung zu übertragen,
eine zweite Gruppe, die dazu eingerichtet ist, die Empfangssignale (33) direkt an die zweite Verzögerungsadditionsschaltung zu übertragen, ohne sie durch die erste Verzögerungsadditionsschaltung laufen zu lassen,
**gekennzeichnet dadurch, dass**
die Ultraschalltransducer der zweiten Gruppe durchgängig in der Umgebung des Mittelpunkts einer Apertur, durch die ein Ultraschallwellenstrahl gebildet wird, angeordnet sind.

2. Ultraschalldiagnosevorrichtung nach Anspruch 1,
wobei die erste Gruppe mehrere der Unteranordnungen (5) enthält, für deren jede die Anzahl der in der Unteranordnung enthaltenen Ultraschalltransducer (1) verschieden voneinander ist.

3. Ultraschalldiagnosevorrichtung nach Anspruch 1,
wobei die in der Unteranordnung (5) der ersten Gruppe enthaltenen Ultraschalltransducer (1) und die in der zweiten Gruppe enthaltenen Ultraschalltransducer alternierend angeordnet sind.

4. Ultraschalldiagnosevorrichtung nach Anspruch 1,
wobei die Ultraschallsonde (100) eine Matrixanordnung bildet, die entlang einer Hauptachse und einer Nebenachse aufgeteilt ist.

5. Ultraschalldiagnosevorrichtung nach Anspruch 1, ferner umfassend:
einen Ultraschalltransducer-Auswahlschalter (70, 71), der dazu ausgelegt ist, zwischen den in der ersten Gruppe enthaltenen Ultraschalltransducern (1) und den in der zweiten Gruppe enthaltenen Ultraschalltransducern beliebig auszuwählen.

## Revendications

1. Appareil de diagnostic par ultrasons comprenant :
une sonde à ultrasons (100) ayant une pluralité de transducteurs ultrasonores (1) dans lequel un nombre prédéterminé parmi la pluralité de transducteurs ultrasonores est configuré comme au moins un sous-ensemble (5, 34, 35), définissant ainsi un premier groupe de transducteurs ultrasonores,
au moins un premier circuit d'addition de retard (13, 36, 37) configuré pour mettre en oeuvre un retard et l'ajouter dans l'au moins un sous-ensemble (5, 34, 35) sur des signaux de réception acquis depuis les transducteurs ultrasonores inclus dans l'au moins un sous-ensemble, et
un deuxième circuit d'addition de retard (31) configuré pour mettre en oeuvre un retard et l'ajouter sur des signaux de réception acquis depuis la pluralité de transducteurs ultrasonores,
dans lequel la pluralité de transducteurs ultrasonores incluent :
le premier groupe configuré pour transmettre les signaux de réception (32), qui sont traités par le premier circuit d'addition de retard (13, 36, 37), au deuxième circuit d'addition de retard, et
un deuxième groupe configuré pour transmettre les signaux de réception (33) directement au deuxième circuit d'addition de retard sans passer par le premier circuit d'addition de retard,
**caractérisé en ce que**
les transducteurs ultrasonores du deuxième groupe sont disposés de manière continue à proximité du centre d'une ouverture à travers laquelle un faisceau d'ondes ultrasonores est formé.

2. Appareil de diagnostic par ultrasons selon la revendication 1,
dans lequel le premier groupe inclut une pluralité des sous-ensembles (5) pour chacun desquels le nombre des transducteurs ultrasonores (1) inclus dans le sous-ensemble est différent les uns des autres.

3. Appareil de diagnostic par ultrasons selon la revendication 1,
dans lequel les transducteurs ultrasonores (1) inclus dans le sous-ensemble (5) du premier groupe et les transducteurs ultrasonores inclus dans le deuxième groupe sont disposés de façon alternée.

4. Appareil de diagnostic par ultrasons selon la revendication 1,
dans lequel la sonde à ultrasons (100) forme une matrice d'éléments qui est divisée le long d'un grand axe et d'un petit axe.

5. Appareil de diagnostic par ultrasons selon la revendication 1, comprenant en outre :
un commutateur (70, 71) de sélection de transducteurs ultrasonores adapté à sélectionner arbitrairement entre les transducteurs ultrasonores (1) inclus dans le premier groupe et les transducteurs ultrasonores inclus dans le deuxième groupe.
